# EUROPEAN PATENT SPECIFICATION

(11) **EP 1 345 628 B1**
(45) Date of publication and mention of the grant of the patent: **13.04.2011**
(21) Application number: 01984811.8
(22) Date of filing: 08.12.2001
(51) Int. Cl.: A61K 47/48

(54) **CONJUGATES OF ERYTHROPOIETIN (EPO) WITH POLYETHYLENE GLYCOL (PEG)**
KONJUGATE VON ERYTHROPOIETIN (EPO) MIT POLYETHYLENGLYKOL (PEG)
CONJUGUES D'ERYTHROPOIETINE (EPO) AVEC POLYETHYLENE GLYCOL (PEG)

(30) Priority: 20.12.2000 EP 00127891
(43) Date of publication of application: 24.09.2003
(73) Proprietor: F. Hoffmann-La Roche AG, 4070 Basel (CH)
(72) Inventor: BURG, Josef, 82362 Weilheim (DE); ENGEL, Alfred, 82327 Tutzing (DE); FRANZE, Reinhard, 82377 Penzberg (DE); HILGER, Bernd, 82377 Penzberg (DE); SCHURIG, Hartmut, Ernst, 81476 Muenchen (DE); TISCHER, Wilhelm, 82380 Peissenberg (DE); WOZNY, Manfred, 82362 Weilheim (DE)
(74) Representative: Waschbüsch, Klaus
(86) International application number: PCT/EP2001/014434
(87) International publication number: WO 2002/049673

(56) References cited:
- EP-A- 1 064 951
- WO-A-00/32772
- WO-A-00/42175
- WO-A-01/02017
- WO-A-01/87329
- WO-A-97/03106
- WO-A-98/32466

### SEQUENCE LISTING

<110> Hoffmann-La Roche AG
   <120> Erythropoietin Conjugates
   <130> Case 20805
   <160> 5
   <170> Patent In version 3.1
<210> 1
   <211> 165
   <212> PRT
   <213> Homo sapiens
<400> 1
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr Ile Thr Ala Asp Thr Phe Arg Lys Leu Phe Arg Val
       130                 135                 140
   Tyr Ser Asn Phe Leu Arg Gly Lys Leu Lys Leu Tyr Thr Gly Glu Ala
   145                 150                 155                 160
   Cys Arg Thr Gly Asp
                   165
<210> 2
   <211> 166
   <212> PRT
   <213> Homo sapiens
<400> 2
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr Ile Thr Ala Asp Thr Phe Arg Lys Leu Phe Arg Val
       130                 135                 140
   Tyr Ser Asn Phe Leu Arg Gly Lys Leu Lys Leu Tyr Thr Gly Glu Ala
   145                 150                 155                 160
   Cys Arg Thr Gly Asp Arg
                   165
<210> 3
   <211> 174
   <212> PRT
   <213> CHO/dhfr-
<400> 3
   Ala Pro Pro Arg Ile Glu Gly Arg Ala Pro Pro Arg Leu Ile Cys Asp
   1               5                   10                  15
   Ser Arg Val Leu Glu Arg Tyr Leu Leu Glu Ala Lys Glu Ala Glu Asn
               20                  25                  30
   Ile Thr Thr Gly Cys Ala Glu His Cys Ser Leu Asn Glu Asn Ile Thr
           35                  40                  45
   Val Pro Asp Thr Lys Val Asn Phe Tyr Ala Trp Lys Arg Met Glu Val
       50                  55                  60
   Gly Gln Gln Ala Val Glu Val Trp Gln Gly Leu Ala Leu Leu Ser Glu
   65                  70                  75                  80
   Ala Val Leu Arg Gly Gln Ala Leu Leu Val Asn Ser Ser Gln Pro Trp
                   85                  90                  95
   Glu Pro Leu Gln Leu His Val Asp Lys Ala Val Ser Gly Leu Arg Ser
               100                 105                 110
   Leu Thr Thr Leu Leu Arg Ala Leu Gly Ala Gln Lys Glu Ala Ile Ser
           115                 120                 125
   Pro Pro Asp Ala Ala Ser Ala Ala Pro Leu Arg Thr Ile Thr Ala Asp
       130                 135                 140
   Thr Phe Arg Lys Leu Phe Arg Val Tyr Ser Asn Phe Leu Arg Gly Lys
   145                 150                 155                 160
   Leu Lys Leu Tyr Thr Gly Glu Ala Cys Arg Thr Gly Asp Arg
                   165                 170
<210> 4
   <211> 169
   <212> PRT
   <213> CHO/dhfr-
<400> 4
   Ala Pro Pro Ala Pro Pro Arg Leu Ile Cys Asp Ser Arg Val Leu Glu
   1               5                   10                  15
   Arg Tyr Leu Leu Glu Ala Lys Glu Ala Glu Asn Ile Thr Thr Gly Cys
               20                  25                  30
   Ala Glu His Cys Ser Leu Asn Glu Asn Ile Thr Val Pro Asp Thr Lys
           35                  40                  45
   Val Asn Phe Tyr Ala Trp Lys Arg Met Glu Val Gly Gln Gln Ala Val
       50                  55                  60
   Glu Val Trp Gln Gly Leu Ala Leu Leu Ser Glu Ala Val Leu Arg Gly
   65                  70                  75                  80
   Gln Ala Leu Leu Val Asn Ser Ser Gln Pro Trp Glu Pro Leu Gln Leu
                   85                  90                  95
   His Val Asp Lys Ala Val Ser Gly Leu Arg Ser Leu Thr Thr Leu Leu
               100                 105                 110
   Arg Ala Leu Gly Ala Gln Lys Glu Ala Ile Ser Pro Pro Asp Ala Ala
           115                 120                 125
   Ser Ala Ala Pro Leu Arg Thr Ile Thr Ala Asp Thr Phe Arg Lys Leu
       130                 135                 140
   Phe Arg Val Tyr Ser Asn Phe Leu Arg Gly Lys Leu Lys Leu Tyr Thr
   145                 150                 155                 160
   Gly Glu Ala Cys Arg Thr Gly Asp Arg
                   165
<210> 5
   <211> 174
   <212> PRT
   <213> CHO/dhfr-
<400> 5
   Ala Pro Pro Gly Ala Ala His Tyr Ala Pro Pro Arg Leu Ile Cys Asp
   1               5                   10                  15
   Ser Arg Val Leu Glu Arg Tyr Leu Leu Glu Ala Lys Glu Ala Glu Asn
               20                  25                  30
   Ile Thr Thr Gly Cys Ala Glu His Cys Ser Leu Asn Glu Asn Ile Thr
           35                  40                  45
   Val Pro Asp Thr Lys Val Asn Phe Tyr Ala Trp Lys Arg Met Glu Val
       50                  55                  60
   Gly Gln Gln Ala Val Glu Val Trp Gln Gly Leu Ala Leu Leu Ser Glu
   65                  70                  75                  80
   Ala Val Leu Arg Gly Gln Ala Leu Leu Val Asn Ser Ser Gln Pro Trp
                   85                  90                  95
   Glu Pro Leu Gln Leu His Val Asp Lys Ala Val Ser Gly Leu Arg Ser
               100                 105                 110
   Leu Thr Thr Leu Leu Arg Ala Leu Gly Ala Gln Lys Glu Ala Ile Ser
           115                 120                 125
   Pro Pro Asp Ala Ala Ser Ala Ala Pro Leu Arg Thr Ile Thr Ala Asp
       130                 135                 140
   Thr Phe Arg Lys Leu Phe Arg Val Tyr Ser Asn Phe Leu Arg Gly Lys
   145                 150                 155                     160
   Leu Lys Leu Tyr Thr Gly Glu Ala Cys Arg Thr Gly Asp Arg
                   165                 170

## Description

Erythropoiesis is the production of red blood cells, which occurs to offset cell destruction. Erythropoiesis is a controlled physiological mechanism that enables sufficient red blood cells to be available for proper tissue oxygenation. Naturally occurring human erythropoietin (EPO) is produced in the kidney and is the humoral plasma factor which stimulates red blood cell production (Carnot, P and Deflandre, C (1906) C.R. Acad. Sci. 143: 432; Erslev, AJ (1953 Blood 8: 349; Reissmann, KR (1950) Blood 5: 372; Jacobson, LO, Goldwasser, E, Freid, W and Plzak, LF (1957) Nature 179: 6331-4). Naturally occurring EPO stimulates the division and differentiation of committed erythroid progenitors in the bone marrow and exerts its biological activity by binding to receptors on erythroid precursors (Krantz, BS (1991) Blood 77: 419).

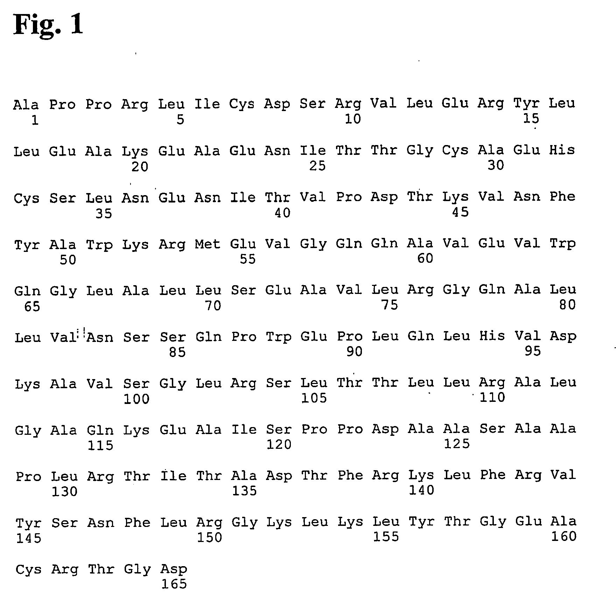

Erythropoietin has been manufactured biosynthetically using recombinant DNA technology (Egrie, JC, Strickland, TW, Lane, J et al. (1986) Immunobiol. 72: 213-224) and is the product of a cloned human EPO gene inserted into and expressed in the ovarian tissue cells of the Chinese hamster (CHO cells). The primary structure of the predominant, fully processed form of human erythropoietin (hEPO) is illustrated in Fig.1. There are two disulfide bridges between Cys⁷-Cys¹⁶¹ and Cys²⁹-Cys³³. The molecular weight of the polypeptide chain of EPO without the sugar moieties is 18,236 Da. In the intact EPO molecule, approximately 40% of the molecular weight are accounted for by the carbohydrate groups that glycosylate the protein at glycosylation sites on the protein (Sasaki, H, Bothner, B, Dell, A and Fukuda, M (1987) J. Biol. Chem. 262: 12059).

Because human erythropoietin is essential in red blood cell formation, the hormone is useful in the treatment of blood disorders characterised by low or defective red blood cell production. Clinically, EPO is used in the treatment of anemia in chronic renal failure patients (CRF) (Eschbach, JW, Egri, JC, Downing, MR et al. (1987) NEJM 316: 73-78; Eschbach, JW, Abdulhsdi, MH, Browne, JK et al. (1989) Ann. Intern. Med. 111: 992; Egrie, JC, Eschbach, JW, McGuire, T, Adamson, JW (1988) Kidney Intl. 33: 262; Lim, VS, Degowin, RL, Zavala, D et al. (1989) Ann. Intern. Med. 110; 108-114) and in AIDS and cancer patients undergoing chemotherapy (Danna, RP, Rudnick, SA, Abels, RI In: MB, Garnick, ed. Erythropoietin in Clinical Applications-An International Perspective. New York, NY: Marcel Dekker; 1990: p. 301-324). However, the bioavailability of commercially available protein therapeutics such as EPO is limited by their short plasma half-life and susceptibility to protease degradation. So, for example, pegylated EPO derivatives have been proposed to overcome these disadvantages.

Common routes to pegylated proteins yield mixtures mono- and oligo-pegylated proteins. Moreover, the polyethylene glycol compound (PEG) is bound at several positions of the proteins depending in the amount and reactivities of the available reactive groups on the protein surface. Such a mixture may result in severe shortcomings: PEG may be bound at positions which interact with the protein specific receptor and conclusively reduce or even prohibit therapeutic efficacy. To solve this drawback either separation and purification of active ingredients of such a mixture or a selective synthetic route to avoid formation is required. Clearly avoiding any formation of mixtures makes it easier to receive a pure active pharmaceutical ingredient in terms of a single positional isomer in essentially higher yields. Separation of positional isomers of PEG-protein mixtures may even be impossible with common tools in production scale.

Examples of PEG-protein mixtures are mentioned in International Patent Applications WO 00/32772, WO 97/03106 and WO 00/42175. WO 00/32772 refers to polymer derivatized non-glycosylated erythropoietic compounds and methods for preparing these proteins involving the use of a linkerless aldehyde modification process. WO 97/03106 discloses poly(ethylene glycol) and related polymers monosubstituted with propionic or butanoic acids and functional derivatives for biotechnical applications, e.g. the use of succimidylesters to produce protein conjugates. WO 00/42175 relates to methods of making soluble proteins having free cysteins in which a host cell is exposed to a cysteine blocking agent. The soluble proteins produced by the methods can then be
modified to increase their effectiveness. Such modifications include attaching a PEG moiety to form pegylated proteins.

Several methods have been proposed for the selective modification of recombinantly produced polypeptides.

European Patent Application EP 651,761 discloses the selective modification of recombinantly produced polypeptides at terminal α-carbon reactive groups. The first step in the method is to form a recombinantly produced polypeptide so that it is protected at the terminal α-carbon reactive group with a biologically added protecting group. The biologically added protecting group is preferably an amino acid, peptide and/or polypeptide that contains at least one site that is cleavable enzymatically or chemically, and preferably has a sequence that is not present in the sequence of the desired polypeptide. Once formed the biologically protected polypeptide is reacted with chemical protecting agents to protect the side chain groups and then is cleaved with a cleavage reagent specific for the biologically added protecting group. By these means a polypeptide is produced having an unprotected N-terminal amino group and protected side chain reactive groups. The unprotected N-terminal amino group is modified with a modifying agent to form an N-terminally modified and side-chain-protected polypeptide. It is then deprotected to form an N-terminally modified polypeptide. EP 651,761 teaches that any sequence of amino acids may be attached as biologically added protecting groups. However, in mammalian expression systems EPO is expressed with a leader signal sequence which is cleaved off by a signal peptidase in order to yield the processed, mature EPO. Such signal peptidases recognise only restricted amino acid residues at the P1' and P3' cleavage site (R. E. Dalbey et al. Protein Sci. 6, 1129 (1997). Conclusively, a biologically added protecting peptide has to be built up from an N-terminal amino acid sequence of at least three amino acids for cleavage of the signal sequence, followed by an amino acid sequence for enzymatic or chemical removal of the protecting group. If the recognition sequences of both the signal peptidase and the cleavage protease are identical or closely related then the sequence of the biologically added protecting group can be reduced to a few amino acids.

Further, N-terminal selective modification was achieved by chemoselective ligation to an aldehyde (or ketone)-functionalized target macromolecule (European Patent Application EP 788,375; Gaertner, HF, Offord, RE, Bioconjugate Chem., 7 (1), 38 -44 (1996)). However, this method only works for N-terminal serines or threonines.

Selective modification at N-terminal alanine was demonstrated by transamination of alanine to pyruvate (European Patent Applications EP 964,702 and EP 605,963). Disadvantage of this method is that the formed EPO derivative showed a reduced in vitro activity. Also the transformation agents Cu²⁺/glyoxylic acid/NaOAc are likely to produce side reactions within the EPO molecule.

Site specific N-terminal modification was also shown by transglutaminase-mediated incorporation of poly(ethylene glycol) derivatives (Sato, H. , Yamamoto, K, Hayashi, E, Takahara, Y, Bioconjugate Chem. 11(4), 502-509 (2000)). But this method showed only low yields and needs incorporation of a peptide tag at the N-terminus and hence modifies the polypeptide structure.

Modification with glyoxylyl-based labelling reagents also enables selective N-terminal modification (Zhao, ZG, Im, JS, Clarke, DF, Bioconjugate Chem. 10, 424 - 430 (1999)) but is restricted to cysteine.

WO 00/32772 discloses pharmaceutical agents for treating anemias by polymer derivatized non-glycosylated erythropoietic compounds which show stability and bioactivity in vivo. Further provided are methods for preparing these derivatived proteins which involve the use of a linkerless aldehyde modification process.

WO 97/03106 discloses active esters of PEG acids and related polymers that have a single propionic or butanoic acid moiety and no ester linkages. Disclosed are also conjugates with proteins, enzymes, polypeptides, drugs, dyes, etc.

WO 98/32466 relates to the attachment of a polyethylene glycol (PEG) moiety to a target molecule. Moreover, a process for direct covalent PEGylation of a substrate, comprising the reaction of halogenated PEG with the substrate wherein the halogen or the halogenated PEG acts as a leaving group in the PEGylation reaction is disclosed.

WO 00/42175 relates to methods of making soluble proteins having free cysteines in which a host cell is exposed to a cysteine blocking agent. The soluble proteins produced by the disclosed methods can be modified to increase their effectiveness. Such modifications include attaching a PEG moiety to form pegylated proteins.

This invention provides an erythropoietin conjugate, said conjugate comprising an erythropoietin glycoprotein having an N-terminal α-amino group and having the *in vivo* biological activity of causing bone marrow cells to increase production of reticulocytes and red blood cells and selected from the group consisting of human erythropoietin and analogs thereof which have sequence of human erythropoietin modified by the addition of from 1 to 6 glycosylation sites or a rearrangement of at least one glycosylation site; said glycoprotein being covalently linked to one poly(ethylene glycol) group of the formula

-CO-(CH₂)ₓ-(OCH₂CH₂)ₘ-OR

with the -CO of the poly(ethylene glycol) group forming an amide bond with said N-terminal α-amino group; wherein R is lower alkyl; x is 2 or 3; and m is from about 450 to about 1350.

Compared to unmodified EPO (i.e., EPO without a PEG attached) and conventional PEG-EPO conjugates, the present conjugates have an increased circulating half-life and plasma residence time, decreased clearance, and increased clinical activity *in vivo.* The conjugates of this invention have the same uses as EPO. In particular, the conjugates of this invention are useful to treat patients by stimulating the division and differentiation of committed erythroid progenitors in the bone marrow in the same way EPO is used to treat patients.

This invention provides conjugates, said conjugate comprising an erythropoietin glycoprotein having an N-terminal α-amino group and having the *in vivo* biological activity of causing bone marrow cells to increase production of reticulocytes and red blood cells and selected from the group consisting of human erythropoietin and analogs thereof which have sequence of human erythropoietin modified by the addition of from 1 to 6 glycosylation sites or a rearrangement of at least one glycosylation site; said glycoprotein being covalently linked to one poly(ethylene glycol) group of the formula

-CO-(CH₂)ₓ-(OCH₂CH₂)ₘ-OR

with the -CO -CO (i.e. carbonyl) of the poly(ethylene glycol) group forming an amide bond with said N-terminal α-amino group; wherein R is lower alkyl; x is 2 or 3; and m is from about 450 to about 1350; i.e. m is chosen so that the molecular weight of the conjugate minus the erythropoietin glycoprotein is from about 20 kDa (kilodaltons) to about 60 kDa.

It has been found that the conjugates of this invention can be used in the same manner as unmodified EPO. However, the conjugates of this invention have an increased circulating half-life and plasma residence time, decreased clearance, and increased clinical activity *in vivo.* Because of these improved properties, the conjugates of this invention can be administered once weekly instead of the three times weekly for unmodified EPO. Decreased frequency of administration is expected to result in improved patient compliance leading to improved treatment outcomes, as well as improved patient quality of life. Compared to conventional conjugates of EPO linked to poly(ethylene glycol) it has been found that conjugates having the molecular weight and linker structure of the conjugates of this invention have an improved potency, stability, area under the curve (AUC), and circulating half-life.

The term "N-terminal α-amino group" refers to the N-terminal amino residue of a peptide, i.e. that end of a peptide or protein chain having an amino acid with a free α-amino (NH₂-) group.

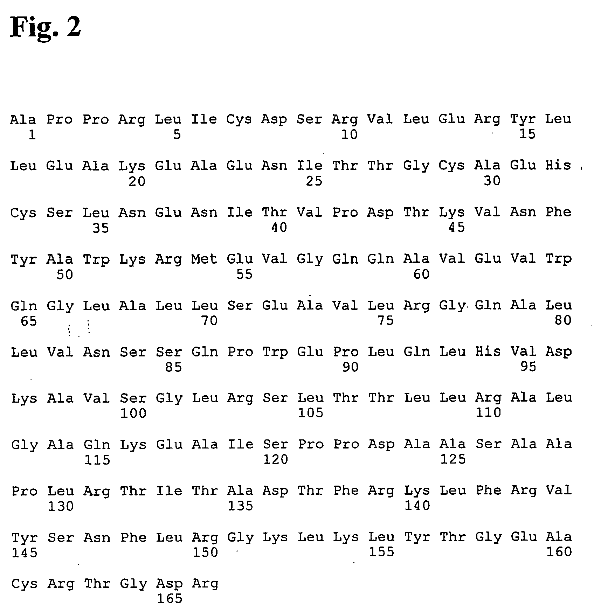

The term "erythropoietin" or "EPO" refers to a glycoprotein, having the amino acid sequence set out in Fig. 1 (SEQ ID NO:1) or Fig. 2 (SEQ ID NO:2), preferably in Fig. 1, or an amino acid sequence substantially homologous thereto, whose biological properties relate to the stimulation of red blood cell production and the stimulation of the division and differentiation of committed erythroid progenitors in the bone marrow. As used herein, these terms include such proteins modified deliberately, as for example, by site directed mutagenesis or accidentally through mutations. These terms also include analogs having from 1 to 6 additional sites for glycosylation, analogs having at least one additional amino acid at the carboxy terminal end of the glycoprotein, wherein the additional amino acid includes at least one glycosylation site, and analogs having an amino acid sequence which includes a rearrangement of at least one site for glycosylation. These terms include both natural and recombinantly produced human erythropoietin.

The term "intermediate EPO" refers to an erythropoietin glycoprotein derivative with a N-terminal extension. Preferably the amino acid extension comprises a secretion signal sequence, optionally followed by a purification tag, e.g. histidine tag (as described for example in H. M. Sassenfeld, Trends in Biotechnol. 8, 88 - 93 (1990)), followed by an enzyme recognition sequence for digestion of a protein which is followed by the erythropoietin glycoprotein amino acid sequence as defined below.

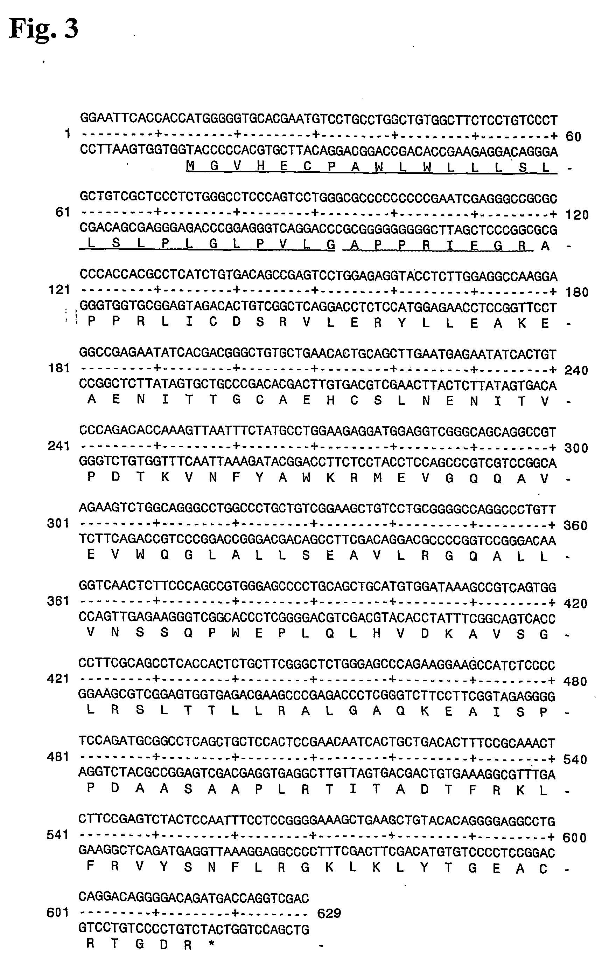

The term "modified EPO" refers to an intermediate EPO wherein the secretion signal has been cleaved off, e.g. to an EPO glycoprotein which is extended at the N-terminus by a proteolytic cleavage site, e.g. the sequence APPRIEGR, i.e. APPRIEGR-EPO (SEQ ID NO:3), or APP, i.e. APP-EPO (SEQ ID NO: 4), or APPGAAHY, i.e. APPGAAHY-EPO (SEQ ID NO:5) or a sequence substantially homologous thereto (see also Figures 3, 4 and 5).

The term "protected modified EPO" refers to a modified EPO, which is received by acylation of ε-amino groups with chemical protecting agents, e.g. by citraconylation.

The term "protected EPO" means the EPO derivative which is obtained after proteolytic cleavage of the protected modified EPO, i.e. EPO wherein the ε-amino groups have been modified with chemical protecting agents and wherein there is a free N-terminal α-amino group.

The term "homologous amino acid sequence" means that the corresponding amino acid sequences have at least 80% amino acid sequence identity with the corresponding proteolytic cleavage sequences or the corresponding erythropoietin amino acids shown in Fig. 1 or 2 and show the required biological activity (either cleavable by the corresponding protease or having the *in vivo* biological activity of causing bone marrow cells to increase production of reticulocytes and red blood cells in the case of the pegylated erythropoietin compounds as defined in the description and claims. Preferably, the homology is 90%, more preferably 95%.

The erythropoietin conjugates of this invention can be represented by Formula 1:

P-NHCO-(CH₂)ₓ-(OCH₂CH₂)ₘ-OR (I)

wherein x, m and R are as above. In Formula I, P is the residue of an erythropoietin glycoprotein described herein, (i.e. without the N-terminal α-amino group which forms an amide linkage with the carbonyl shown in Formula I), having the *in vivo* biological activity of causing bone marrow cells to increase production of reticulocytes and red blood cells.

In a preferred embodiment of the present invention R is methyl. Preferably, m is from about 550 to about 1000, more preferably from about 650 to about 750.

In the most preferred embodiment of the present invention R is methyl, and m is from about 650 to about 750, i.e. the conjugate as defined above having the formula

CH₃O(CH₂CH₂O)ₘCH₂CH₂CH₂CO-NH-P

wherein m is from 650 to 750 and P is as defined above. Preferably m has an average of about 730.

Preferably, the glycoprotein of the conjugates as defined above is a human erythropoietin. Human erythropoietin and analogous proteins as defined above can be expressed by endogenous gene activation. Preferred human erythropoietin glycoproteins are those shown in Fig. 1 or 2, most preferably those shown in Fig. 1.

Further, P may be selected from the group consisting of residues of human erythropoietin and analogs thereofhaving from 1 to 6 additional sites for glycosylation. As set out in detail below, the preparation and purification of EPO are well known in the art. By EPO is meant the natural or recombinant protein, preferably human, as obtained from any conventional source such as tissues, protein synthesis, cell culture with natural or recombinant cells. Any protein having the activity of EPO, such as muteins or otherwise modified proteins, is encompassed. Recombinant EPO may be prepared via expression in CHO, BHK, COS, HeLa or PER C6 cell lines or other appropriate cell lines of animal or human origin, by recombinant DNA technology or by endogenous gene activation. Expression of proteins, including EPO, by endogenous gene activation is well known in the art and is disclosed, for example in U.S. Patents Nos. 5,733,761, 5,641,670, and 5,733,746, and international patent publication Nos. WO 93/09222, WO 94/12650, WO 95/31560, WO 90/11354, WO 91/06667 and WO 91/09955.

The preferred EPO species for the preparation of erythropoietin glycoprotein products are human EPO species. More preferably, the EPO species is the human EPO having the amino acid sequence set out in Fig. 1 or Fig. 2, more preferably the amino acid sequence set out in Fig. 1.

In an embodiment, P may be the residue of a glycoprotein analog having from 1 to 6 additional sites for glycosylation. Glycosylation of a protein, with one or more oligosaccharide groups, occurs at specific locations along a polypeptide backbone and greatly affects the physical properties of the protein such as protein stability, secretion, subcellular localisation, and biological activity. Glycosylation is usually of two types. O-linked oligosaccharides are attached to serine or threonine residues and N-linked oligosaccharides are attached to asparagine residues. One type of oligosaccharide found on both N-linked and O-linked oligosaccharides is N-acetylneuraminic acid (sialic acid), which is a family of amino sugars containing 9 or more carbon atoms. Sialic acid is usually the terminal residue on both N-linked and O-linked oligosaccharides and, because it bears a negative charge, confers acidic properties to the glycoprotein. Human erythropoietin, having 165 amino acids, contains three N-linked and one O-linked oligosaccharide chains which comprise about 40% of the total molecular weight of the glycoprotein. N-linked glycosylation occurs at asparagine residues located at positions 24, 38, and 83 and O-linked glycosylation occurs at a serine residue located at position 126. The oligosaccharide chains are modified with terminal sialic acid residues. Enzymatic removal of all sialic acid residues from the glycosylated erythropoietin results in loss of *in vivo* activity but not in vitro activity because sialylation of erythropoietin prevents its binding, and subsequent clearance, by hepatic binding protein.

The glycoproteins of the present invention include analogs of human erythropoietin with one or more changes in the amino acid sequence of human erythropoietin which result in an increase in the number of sites for sialic acid attachment. These glycoprotein analogs may be generated by site-directed mutagenesis having additions, deletions, or substitutions of amino acid residues that increase or alter sites that are available for glycosylation. Glycoprotein analogs having levels of sialic acid greater than those found in human erythropoietin are generated by adding glycosylation sites which do not perturb the secondary or tertiary conformation required for biological activity. The glycoproteins of the present invention also include analogs having increased levels of carbohydrate attachment at a glycosylation site which usually involve the substitution of one or more amino acids in close proximity to an N-linked or O-linked site. The glycoproteins of the present invention also include analogs having one or more amino acids extending from the carboxy terminal end of erythropoietin and providing at least one additional carbohydrate site. The glycoproteins of the present invention also include analogs having an amino acid sequence which includes a rearrangement of at least one site for glycosylation. Such a rearrangement of glycosylation site involves the deletion of one or more glycosylation sites in human erythropoietin and the addition of one or more non-naturally occurring glycosylation sites. Increasing the number of carbohydrate chains on erythropoietin, and therefore the number of sialic acids per erythropoietin molecules may confer advantageous properties such as increased solubility, greater resistance to proteolysis, reduced immunogenecity, increased serum half-life, and increased biological activity. Erythropoietin analogs with additional glycosylation sites are disclosed in more detail in European Patent Application 640 619, to Elliot published March 1,1995.

In a preferred embodiment, the glycoproteins of the present invention comprise an amino acid sequence which includes at least one additional site for glycosylation such as, but not limited to, erythropoietins comprising the sequence of human erythropoietin modified by a modification selected from the following:
Asn³⁰Thr³²;
Asn⁵¹Thr⁵³,
Asn⁵⁷Thr⁵⁹;
Asn⁶⁹;
Asn⁶⁹Thr⁷¹;
Ser⁶⁸Asn⁶⁹Thr⁷¹;
Val⁸⁷Asn⁸⁸Thr⁹⁰;
Ser⁸⁷Asn⁸⁸Thr⁹⁰;
Ser⁸⁷Asn⁸⁸Gly⁸⁹Thr⁹⁰;
Ser⁸⁷Asn⁸⁸Thr⁹⁰Thr⁹²;
Ser⁸⁷Asn⁸⁸Thr⁹⁰Ala¹⁶²;
Asn⁶⁹Thr⁷¹Ser⁸⁷Asn⁸⁸Thr⁹⁰;
Asn³⁰Thr³²Val⁸⁷Asn⁸⁸Thr⁹⁰;
Asn⁸⁹Ile⁹⁰Thr⁹¹;
Ser⁸⁷Asn⁸⁹Ile⁹⁰Thr⁹¹;
Asn¹³⁶Thr¹³⁸;
Asn¹³⁸Thr¹⁴⁰;
Thr¹²⁵; and
Pro¹²⁴Thr¹²⁵.

The notation used herein for modification of amino acid sequence means that the position(s) of the corresponding unmodified protein (e.g. hEPO of Fig. 1 and Fig. 2) indicated by the superscripted number(s) is changed to the amino acid(s) that immediately precede the respective superscripted number(s).

The glycoprotein may also be an analog having an amino acid sequence which includes a rearrangement of at least one site for glycosylation. The rearrangement may comprise a deletion of any of the N-linked carbohydrate sites in human erythropoietin and an addition of an N-linked carbohydrate site at position 88 of the amino acid sequence of human erythropoietin. Preferably, the glycoprotein is an analog selected from the group consisting of Gln²⁴ Ser⁸⁷ Asn⁸⁸ Thr⁹⁰ EPO; Gln³⁸ Ser⁸⁷ Asn⁸⁸ Thr⁹⁰ EPO; and Gln⁸³ Ser⁸⁷ Asn⁸⁸ Thr⁹⁰ EPO.

As used herein, "lower alkyl" means a linear or branched alkyl group having from one to six carbon atoms. Examples of lower alkyl groups include methyl, ethyl and isopropyl. In accordance with this invention, R is any lower alkyl. Conjugates in which R is methyl are preferred.

The symbol "m" represents the number of ethylene oxide residues (OCH₂CH₂) in the poly(ethylene oxide) group. A single PEG subunit of ethylene oxide has a molecular weight of about 44 daltons. Thus, the molecular weight of the conjugate (excluding the molecular weight of the EPO) depends on the number "m". In the conjugates of this invention "m" is from about 450 to about 1350 (corresponding to a molecular weight of about 20 kDa to about 60 kDa), preferably from about 650 to about 750 (corresponding to a molecular weight of about 30 kDa), most preferably about 730. The number m is selected such that the resulting conjugate of this invention has a physiological activity comparable to unmodified EPO, which activity may represent the same as, more than, or a fraction of the corresponding activity of unmodified EPO. A molecular weight of "about" a certain number means that it is within a reasonable range of that number as determined by conventional analytical techniques. The number "m" is selected so that the molecular weight of the poly(ethylene glycol) chain covalently linked to the erythropoietin glycoprotein is from about 20 kDa to about 60 kDa, and is preferably about 32 kDa.

The steps of the method for the preparation of the above compounds involve forming a recombinant single copy erythropoietin glycoprotein or a portion thereof so that the single copy glycoprotein is protected with one or more biologically added protecting groups at the N-terminal α-amine. The recombinant erythropoietin can than be reacted with a protecting agent to selectively protect reactive side chain amino groups and thereby prevent amino side chains groups from being modified with the pegylation reagent. The erythropoietin glycoprotein can be cleaved with at least one cleavage reagent specific for the biological protecting group to form an unprotected terminal amino acid α-carbon reactive amino group. The unprotected terminal amino acid α-carbon reactive group may be modified with a pegylation reagent. The side chain protected terminally modified erythropoietin glycoprotein is then deprotected at the side chain groups to form a terminally modified (= pegylated) recombinant erythropoietin glycoprotein.

Therefore, the invention also refers to a process comprising
a) expression and, preferably serumfree fermentation, of a recombinant EPO protein comprising a N-terminal peptidic extension which comprises a proteolytic cleavage site
b) protecting the ε-amino groups,
c) proteolytic cleavage of the N-terminal peptidic extension,
d) pegylation of the N-terminal ε-amino group,
e) deprotection of the ε-amino groups of the erythropoietin glycoprotein,
f) wherein optionally each of the above steps may be followed by a purification step.

The invention also relates to the above process wherein the recombinant EPO comprises a sequence selected from the group consisting of the amino acid sequences shown in Figures 1 to 5. The ε-amino groups may be protected by a citraconylation and the N-terminal α-amino group may be pegylated with 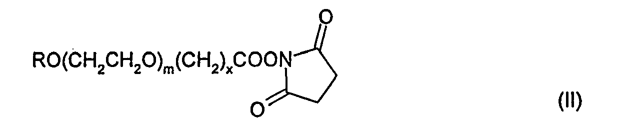 in which R, m and x are as defined above.

In more detail, the above steps may be performed as follows:

### A) Expression, fermentation and purification of modified EPO :

Cloning and expression methods for EPO and EPO related molecule are known in the art. Human erythropoietin (EPO) is a human glycoprotein which stimulates the formation of erythrocytes. Its preparation and therapeutic application are described in detail for example in U.S. Patent Nos. 5,547,933 and 5,621,080, EP-B 0 148 605, Huang, S.L., Proc. Natl. Acad. Sci. USA (1984) 2708-2712, EP-B 0 205 564, EP-B 0 209 539 and EP-B 0 411 678 as well as Lai, P.H. et al., J. Biol. Chem. 261 (1986) 3116-3121, an Sasaki, H. et al., J. Biol. Chem. 262 (1987) 12059-12076. Erythropoietin for therapeutic uses may be produced by recombinant means (EP-B 0 148 605, EP-B 0 209 539 and Egrie, J.C., Strickland, T.W., Lane, J. et al. (1986) Immunobiol. 72: 213-224).

Methods for the expression and preparation of erythropoietin in serum free medium are described for example in WO 96/35718, to Burg published 14 November 1996, and in European Patent Publication No. 513 738, to Koch published 12 June 1992. In addition to the publications mentioned above, it is known that a serum-free fermentation of recombinant CHO cells which contain an EPO gene can be carried out. Such methods are described for example in EP-A 0 513 738, EP-A 0 267 678 and in a general form by Kawamoto, T. et al., Analytical Biochem. 130 (1983) 445-453, EP-A 0 248 656, Kowar, J. and Franek, F., Methods in Enzymology 421 (1986) 277-292, Bavister, B., Exp. Zoology 271 (1981) 45-51, EP-A 0 481 791, EP-A 0 307 247, EP-A 0 343 635, WO 88/00967.

Purification methods for the erythropoietin and derivatives thereof are also known in the art:

In EP-A 0 267 678 an ion exchange chromatography on S-Sepharose, a preparative reverse phase HPLC on a C₈ column and a gel filtration chromatography are described for the purification of EPO produced in serum-free culture after dialysis. In this connection the gel filtration chromatography step can be replaced by ion exchange chromatography on S-Sepharose fast flow. It is also proposed that a dye chromatography on a Blue Trisacryl column be carried out before the ion exchange chromatography.

A process for the purification of recombinant EPO is described by Nobuo, I. et al., J. Biochem. 107 (1990) 352-359. In this process EPO is treated however with a solution of Tween^{®} 20, phenylmethylsulfonyl fluoride, ethylmaleimide, pepstatin A, copper sulfate and oxamic acid prior to the purification steps. Publications, including WO 96/35718, to Burg published 14 November 1996, discloses a process for preparing erythropoietin in a serum free fermentation process (EPOsf).

### B) Reaction with a protecting agent to selectively protect reactive side chain amino groups: Preparation of protected modified EPO

Suitable chemical protecting agents form bonds at unprotected side chain amines and are less stable than and different from those bonds at the N-terminus. Plenty of chemical protecting agents are known (see for example European Patent Application EP 651,761). Preferred are cyclic dicarboxylic acid anhydrides like maleic or citraconylic anhydrides.

Citraconylation is the preferred method if the target polypeptide or fusion protein (herein termed modified EPO) properties accept slightly alkaline conditions for protection and acidic conditions for deprotection (Dixon, HBF; Perham, RN : Biochem. J. 109(2), 312 - 14 (1968); Atassi, MZ, Habeeb, AFSA: Methods Enzymol. 25(Pt. B), 546 - 53 (1972)).

Optionally the protected modified EPO may be purified before performing the next step.

### Proteolytic cleavage of protected modified EPO: Preparation of protected EPO

Suitable proteases for cleavage of fusion proteins are described by Carter, P: Site-specific proteolysis of fusion proteins, in Protein Purification: From Molecular Mechanisms to Large Scale Processes, ACS, Washington DC, pp. 181 - 193 (1990). Such proteases require a narrow specificity to cleave selectively at their recognition sequence and not anywhere in the target protein sequence. Examples are factor Xa which cleaves at IEGR↓ and enterokinase which cleaves at DDDDK↓. Moreover enterokinase is reported to cleave DDDDK↓AP which indicates specificity at the P1' and P2' site for interleukins (P. Carter). Enterokinase is, however, not preferred if chemical protecting agents to protect the side chain ε-amino groups of lysines have to be introduced. In this case the enzyme would not work any more at the wanted cleaving site.

Among others IgA protease is of use which cleaves preferentially at PP↓XP (X = T, S, A). The XP sequence makes it suitable for interleukins and erythropoietin (EP513073). Another suitable protease is a subtilisin BPN' variant (Genenase™, Genencor Int. Inc.) which cleaves at HY↓.

Optionally the protected EPO protein may be purified at this stage.

### D) Modification with a pegylation reagent:

Human EPO contains nine free amino groups, the amino-terminal amino group plus the ε-amino groups of 8 lysine residues. When the pegylation reagent is a SBA compound of Formula II, it has been found that at pH 7.5, a protein:PEG ratio of 1:3, and a reaction temperature of from 20-25°C, a mixture of mono-, di-, and trace amounts of the tri-pegylated species are produced if it is reacted with EPO. The pegylated EPO can be administered as a mixture, or as the cation exchange chromatography separated different pegylated species. By manipulating the reaction conditions (e.g., ratio of reagents, pH, temperature, protein concentration, time of reaction etc.), the relative amounts of the different pegylated species can be varied.

Using the procedures as specified herein for protected EPO only the N-terminal α-amino group of the N-terminal alanine of the protected EPO is pegylated. Because of protection of all of the ε-amino groups of the lysine side chains neither di-, nor oligo-pegylated protected EPOs are formed

The compound of Formula I can be prepared from the known polymeric material: 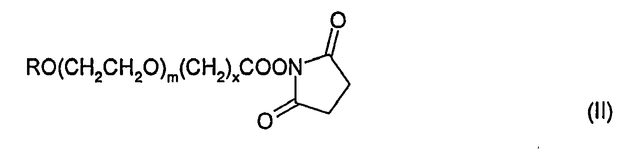 in which R and m are as described above, by condensing the compound of Formula II with the erythropoietin glycoprotein of step c). Compounds of Formula II in which x is 3 are alpha-lower alkoxy, butyric acid succinimidyl esters of poly(ethylene glycol) (lower alkoxy-PEG-SBA). Compounds of Formula II in which x is 2 are alpha-lower alkoxy, propionic acid succinimidyl esters of poly(ethylene glycol) (lower alkoxy-PEG-SPA). Any conventional method of reacting an activated ester with an amine to form an amide can be utilised. In the reaction described above, the exemplified succinimidyl ester is a leaving group causing the amide formation. The use of succinimidyl esters such as the compounds of formula II to produce conjugates with proteins are disclosed in U.S. Patent No. 5,672,662, issued September 30, 1997 (Harris, et al.).

The pegylation reaction may be performed at a molar ratio of 1:5 (EPO to PEG-SBA reagent) up to a final protein concentration of 5 mg/ml. The preferred pegylation reagent is a methoxy-PEG-SBA, which is a compound of Formula II in which R is methyl; x is 3; and m is from 650 to 750 (average about 730, corresponding to an average molecular weight of about 32 kDa, methoxy-PEG-SBA is commercially available: Shearwater Polymers, Inc.).

Purification of the reaction product from the reaction mixture may be achieved by conventional chromatographic purification as described in the Examples.

### E) Deprotection of ε-amino groups (side chain amino groups):

Cleavage of the protection agents may be achieved by conventional methods (see above). In case of decitraconylation, deprotection of the protein may be achieved by stirring the solution at a low pH, e.g. 2.5 for 5 h at ambient temperature. The reaction may be stopped by adjusting the pH to 4.5 with sodium hydroxide and the solution is stored frozen at -20°C until ready for purification.

Purification of the reaction product from the reaction mixture may be achieved by conventional chromatographic purification as described in the Examples.

The specific activity of EPO or EPO conjugates in accordance with this invention can be determined by various assays known in the art. The biological activity of the purified EPO proteins of this invention are such that administration of the EPO protein by injection to human patients results in bone marrow cells increasing production of reticulocytes and red blood cells compared to non-injected or control groups of subjects. The biological activity of the EPO proteins, or fragments thereof, obtained and purified in accordance with this invention can be tested by methods according to Annable, et al., Bull. Wld. Hlth. Org. (1972) 47: 99-112 and Pharm. Europa Spec. Issue Erythropoietin BRP Bio 1997(2). Another biological assay for determining the activity of EPO protein, the normocythaemic mouse assay, is described in Example 4.

The conjugates in accordance of this invention can be administered in a therapeutically effective amount to patients in the same way EPO is administered. The therapeutically effective amount is that amount of conjugate necessary for the *in vivo* biological activity of causing bone marrow cells to increase production of reticulocytes and red blood cells. The exact amount of conjugate is a matter of preference subject to such factors as the exact type of condition being treated, the condition of the patient being treated, as well as the other ingredients in the composition. For example, 0.01 to 10 µg per kg body weight, preferably 0.1 to 3 µg per kg body weight, may be administered e.g. once weekly.

The invention also refers to the corresponding pharmaceutical compositions comprising a conjugate as described above and a pharmaceutically acceptable excipient.

The pharmaceutical compositions containing the conjugate may be formulated at a strength effective for administration by various means to a human patient experiencing blood disorders characterised by low or defective red blood cell production. Average therapeutically effective amounts of the conjugate may vary and in particular should be based upon the recommendations and prescription of a qualified physician.

The erythropoietin glycoprotein products prepared in accordance with this invention may be prepared in pharmaceutical compositions suitable for injection with a pharmaceutically acceptable carrier or vehicle by methods known in the art. For example, appropriate compositions have been described in WO97/09996, WO97/40850, WO98/58660, and WO99/07401. For example, the compounds of the present invention may be formulated in 10 mM sodium/potassium phosphate buffer at pH 7 containing a tonicity agent, e.g. 132 mM sodium chloride. Optionally the pharmaceutical composition may contain a preservative. The pharmaceutical composition may contain different amounts of erythropoietin, e.g. 10 -10000 µg/ml, e.g. 50 µg/ml or 400 µg/ml.

Preferably, the pharmaceutical composition comprises a conjugate as defined above, a multiple charged inorganic anion in a pharmaceutically acceptable buffer suitable to keep the solution pH in the range from about 5.5 to about 7.0, and optionally one or more pharmaceutically acceptable excipients, e.g. the composition comprises about 10 µg to about 10000 µg erythropoietin conjugate per ml, 10 - 200 mmol/l sulfate, about 10 to about 50 mmol/l phosphate pH 6.0 to 6.5, optionally up to 1 mM CaCl₂ and optionally about 1- 5 % of a polyol. Examples for suitable compositions are:
a) 50 µg/ml or 400 µg/ml erythropoietin conjugate, 10 mM sodium/potassium phosphate, 100 mM NaCl, pH 7.0,
b) 50 µg/ml or 400 µg/ml erythropoietin conjugate, 10 mM sodium phosphate, 120 mM sodium sulfate, pH 6.2,
c) 50 µg/ml or 400 µg/ml erythropoietin conjugate, 10 mM sodium phosphate, 40 mM sodium sulfate, 3% mannitol, pH 6.2,
d) 50 µg/ml or 400 µg/ml erythropoietin conjugate, 10 mM sodium phosphate, 40 mM sodium sulfate, 3% mannitol, 7.5 µM CaCl₂, pH 6.2,
e) 50 µg/ml or 400 µg/ml erythropoietin conjugate, 50 mM arginine, 100 mM sodium sulfate, 1 mM CaCl₂, pH 6.2, and
f) 50 µg/ml or 400 µg/ml erythropoietin conjugate, 50 mM arginine, 30 mM sodium sulfate, 3% mannitol, 1 mM CaCl₂, pH 6.2.

Further preferred composition may comprise 10 to 10000 µg/ml erythropoietin, preferably 25 to 2500 µg/ml erythropoietin, and
a) 10 mM sodium/potassium phosphate, 100 mM NaCl, pH 7.0 or
b) 10 mM sodium phosphate, 120 mM sodium sulfate, pH 6.2 or
c) 10 mM sodium phosphate, 40 mM sodium sulfate, 3% mannitol (w/v), pH 6.2 or
d) 10 mM sodium phosphate, 40 mM sodium sulfate, 3% mannitol (w/v), 10 mM methionine, 0.01% pluronic F68 (w/v), pH 6.2 or
e) 40 mM arginine, 30 mM sodium sulfate, 3% mannitol (w/v), pH 6.2 or
f) 40 mM arginine, 30 mM sodium sulfate, 3% mannitol (w/v), 10 mM methionine, 0.01% pluronic F68 (w/v), pH 6.2.

In the most preferred embodiment, the compositions comprise an amount erythropoietin protein of 50, 100, 400, 800 or 2500 µg/ml. The most preferred compositions comprise either 10 mM sodium phosphate, 40 mM sodium sulfate, 3% mannitol (w/v), 10 mM methionine, 0.01% pluronic F68 (w/v), pH 6.2 or 40 mM arginine, 30 mM sodium sulfate, 3% mannitol (w/v), 10 mM methionine, 0.01% pluronic F68 (w/v), pH 6.2.

The conjugates of the present invention are especially useful for the preparation of medicaments for the treatment or prophylaxis of diseases correlated with anemia in chronic renal failure patients (CRF), AIDS and for the treatment of cancer patients undergoing chemotherapy.

The invention also refers to the use of a conjugate as defined above for the preparation of medicaments, especially for the preparation of medicaments for the treatment or prophylaxis of diseases correlated with anemia in chronic renal failure patents (CRF), AIDS and for the treatment of cancer patients undergoing chemotherapy.

An additional embodiment of the present invention refers to a method for the prophylactic and/or therapeutic treatment of disorders involving anemia in chronic renal failure patients (CRF), AIDS and cancer patients undergoing chemotherapy comprising the step of administering to a patient a conjugate as described above.

The invention refers also to compounds as defined above for the treatment of diseases which are associated with anemia in chronic renal failure patients (CRF), AIDS and cancer patients undergoing chemotherapy.

Another aspect of the present invention comprises the above compounds whenever prepared by a process as described above.

A further embodiment of the invention refers to erythropoietin glycoproteins comprising the amino acid sequences as shown in Fig. 1 and Fig. 2 having a N-terminal peptidic extension which represents a proteolytic cleavage site, optionally comprising a N-terminal purification tag. Examples for these peptides are APPRIEGR-EPO, APP-EPO and APPGAAHY-EPO (see also Fig. 3 to 5). One embodiment of the invention refers to erythropoietin glycoproteins comprising the amino acid sequences as shown in Figures 3 to 5.

The invention will be better understood by reference to the following examples which illustrate but do not limit the invention described herein.

### EXAMPLES

### EXAMPLE 1: Expression, fermentation and purification of modified EPO

### (1) Expression of modified-EPO Constructs

### a) Reagents

Unless specified, all biochemical reagents used were from Roche Diagnostics GmbH (Mannheim, Germany), and all cell culture reagents from Gibco-BRL (Eggenstein, Germany).

### b) Cloning of the wild type EPO expression construct.

For stable expression in Chinese Hamster Ovary (CHO) cells, standard eukaryotic expression vectors such as pcDNA3 (Invitrogen BV, Groningen, Netherlands), pCI-neo (Promega, Madison, WI, USA) were modified by substituting the *neo* gene coding for G418-resistance with the gene coding for mice dehydrofolate reductase (DHFR, Crouse et al. J. Biol. Chem. 257, 7887 - 7897 (1982)), whose expression level is controlled by the simian virus 40 (SV40) early promoter and its late polyadenylation signal. In case of pcDNA3 the resulting vector was designated p. 11381 (M. Tacke et al. Hepatology 26, 1626 - 1633 (1997)).

The wild type erythropoietin coding fragment is obtainable according to methods known in the art, e.g. as described by Jacobs K. et al., Nature 313, 806-10 (1985).
Preferably, the coding fragment is amplified using primers EPO-EcoRI 5'-GAGCCTGAATTCACCACC and EPO-SalI 5'-AGGTGGGTCGACCTGGTCATCTGTCCCCTG. The PCR fragment was digested with EcoRI and SalI (sites are underlined in primer sequences) and cloned into the multiple cloning site of the pre-digested pCI-dhfr vector fragment. Expression of the EPO gene is therefore under control of the human cytomegalovirus (CMV) immediate-early enhancer/promoter region, an optimized chimeric intron for regulated expression and the SV40 late polyadenylation signal.

### c) Cloning of APPRIEGR-EPO expression construct

The APPRIEGR peptide was assembled as a NarI DNA fragment by two annealed oligonucleotides APPRIEGRfor, 5'-CGCCCCCCCCCGAATCGAGGGCCG, and APPRIEGRrev, 5'-CGCGGCCCTCGATTCGGGGGGGGG (remnants of NarI site underlined), and cloned in between the N-terminal signal sequence and the mature EPO coding region.

### d) Cloning of APP-EPO expression construct

The APP peptide was assembled as a NarI DNA fragment by two annealed oligonucleotides APPfor, 5'-CGCCCCCCC, and APPrev, 5'-CGGGGGGGG (remnants of NarI site underlined), and cloned in between the N-terminal signal sequence and the mature EPO coding region.

### e) Cloning of APPGAAHY-EPO expression construct

The APPGAAHY peptide was assembled as a NarI DNA fragment by two annealed oligonucleotides APPGAAHYfor, 5'-CGCCCCCCCCGGCGCCGCCCACTA, and APPGAAHYrev, 5'-CGTAGTGGGCGGCGCCGGGGGGGG (remnants of NarI site underlined), and cloned in between the N-terminal signal sequence and the mature EPO coding region.

### f) Cell culture procedures

The mutagenized cell line CHO/dhfr- (ATCC CRL-9096) deficient in the *dhfr* enzyme gene was obtained from the American Type Tissue Collection (Manassas, VA, USA). Untransfected cells were cultured in α-MEM, 5% dialyzed fetal calf serum (FCS), 2 mM glutamine. Cells were transfected with the EPO plasmids using the FuGENE 6 transfection reagent. Transfected cells were selected in α-MEM lacking nucleosides (α-MEM), supplemented with 10% dialysed FCS, 2 mM glutamine. Single colonies were isolated by FACS, expanded, and the culture supernatants were assayed for production and secretion of EPO by enzyme linked immunosorbant essay (ELISA). The EPO expression levels were enhanced several times by the amplification of the *dhfr* and EPO genes in culture media containing increased concentrations of methotrexate (MTX, Sigma Chemical Co., St. Louis, MO, USA).

### (2) Fermentation

In the following the fermentation and purification of a modified EPO is described.

### Inoculum Preparation and Fermentation

One vial of the Cell Bank, originating from an modified EPO-producing CHO cell line (Host cell line: ATCC CRL-9096, deficient in the *dhfr* enzyme gene) is taken from the vapour phase of the liquid nitrogen storage tank. The cells are transferred into glass spinner flasks and cultivated in a hydrogen carbonate-buffered medium in a humidified CO₂ incubator. Typical serum free media used for the inocolum preparation and fermentation are disclosed in European Patent Application 513 738, to Koch published 12 June 1992, or WO 96/35718, to Burg published 14 November 1996, for example contain as medium DMEM/F12 (e.g. JRH Biosciences/Hazleton Biologics, Denver, US, order No. 57-736) and additionally sodium hydrogencarbonate, L+glutamine, D+glucose, recombinant insulin, sodium selenite, diaminobutane, hydrocortisone, iron(II) sulfate, asparagine, aspartic acid, serine and a stabilizer for mammalian cells such as e.g. polyvinyl alcohol, methyl cellulose, polydextran, polyethylene glycol, Pluronic F68, plasma expander polygelin (HEMACCEL^{®}) or polyvinyl pyrrolidone (WO 96/35718).

The cultures are microscopically checked for the absence of contaminating microorganisms, and the cell densities are determined. These tests are performed at each splitting step.

After the initial growth period, the cell culture is diluted with fresh medium to the starting cell density and undergoes another growth cycle. This procedure is repeated until a culture volume of approximately 21 per glass spinner flask has been obtained. After approx. 12 doublings 1 to 5 litre of this culture is available which then is used as inoculum for the 101 inoculum fermenter.

After 3 - 5 days, the culture in the 101 fermenter can be used as inoculum for the 100 1 inoculum fermenter.

After additional 3 - 5 days of cultivation, the culture in the 1001 fermenter can be used as inoculum for a 10001 production fermenter.

### Harvesting and Cell Separation

A batch refeed process is used, i.e. when the desired cell density is reached, approx. 80 % of the culture is harvested. The remaining culture is replenished with fresh culture medium and cultivated until the next harvest. One production run consists of a maximum of 10 subsequent harvests: 9 partial harvests and 1 overall harvest at the end of fermentation. Harvesting takes place every 3 - 4 days.

The determined harvest volume is transferred into a cooled vessel. The cells are removed by centrifugation or filtration and discarded. The modified EPO containing supernatant of the centrifugation step is in-line filtered and collected in a second cooled vessel. Each harvest is processed separately during purification. A typical purification process of modified EPO-protein is explained in the following.

### (3) Purification of the modified EPOs

A typical process for the purification of EPO-protein is disclosed in WO 96/35718, to Burg published 14 November 1996. The purification process is applicable to modified EPOs and explained in the following:

### (1) Blue Sepharose Chromatography

Blue Sepharose (Pharmacia) consists of Sepharose beads to the surface of which the Cibacron blue dye is covalently bound. Since modified EPO binds more strongly to Blue Sepharose than most non-proteinaceous contaminants and proteinaceous impurities, modified EPO can be enriched in this step. The elution of the Blue Sepharose column is performed by increasing the salt concentration as well as the pH.

The column is filled with Blue Sepharose, regenerated with NaOH and equilibrated with equilibration buffer (sodium/ calcium chloride and sodium acetate). The acidified and filtered fermenter supernatant is loaded. After completion of the loading, the column is washed first with a buffer similar to the equilibration buffer containing a higher sodium chloride concentration and consecutively with a Tris-base buffer. The product is eluted with a Tris-base buffer containing 1 M NaCl and collected in a single fraction.

### (2) Butyl Toyopearl Chromatography

The Butyl Toyopearl 650 C (Toso Haas) is a polystyrene based matrix to which aliphatic butyl-residues are covalently coupled. Since modified EPO binds more strongly to this gel than most of the impurities, it can be eluted with a buffer containing isopropanol.

The column is packed with Butyl Toyopearl 650 C, regenerated with NaOH, washed with a Tris-base buffer and equilibrated with a Tris-base buffer containing isopropanol.

The Blue Sepharose eluate is adjusted to the concentration of isopropanol in the column equilibration buffer and loaded onto the column. Then the column is washed with equilibration buffer with increased isopropanol concentration. The product is eluted with elution buffer (Tris-base buffer with high isopropanol content) and collected in a single fraction.

### (3) Hydroxyapatite Ultrogel Chromatography

The Hydroxyapatite Ultrogel™ (Biosepra) consists of hydroxyapatite which is incorporated in an agarose matrix to improve the mechanical properties, modified EPO has a low affinity to hydroxyapatite and can therefore be eluted at lower phosphate concentrations than protein impurities.

The column is filled with Hydroxyapatite Ultrogel and regenerated with a potassium phosphate/ calcium chloride buffer and NaOH followed by a Tris-base buffer. Then it is equilibrated with a Tris-base buffer containing a low amount of isopropanol and sodium chloride.

The modified EPO containing eluate of the Butyl Toyopearl chromatography is diluted with Tris-base buffer and loaded onto the column. Subsequently the column is washed with equilibration buffer and a Tris-base buffer without isopropanol and sodium chloride. The product is eluted with a Tris-base buffer containing a low concentration of potassium phosphate and collected in a single fraction.

The eluate of the Hydroxyapatite Ultrogel column is concentrated and diafiltered against citraconylation buffer. Concentration/diafiltration is performed with a Millipore Labscale^{™} TFF System fitted with 10 kDa cut off Millipore Pellicon XL membrane.

### EXAMPLE 2: Citraconylation of modified EPO, proteolytic cleavage and purification of the protected EPO.

The solution of the modified EPO is adjusted to pH 8,5 - 9.0 and stirred at room temperature. Citraconic anhydride (Merck 8.41321.0100) is added slowly to the stirred solution in aliquots; pH of 9.0 is maintained by addition of 0,5 N NaOH with a pH-stat. The total amount of citraconic anhydride corresponds to a 5-fold molar excess to the ε-amino groups of lysines in the modified EPO. When the addition of citraconic anhydride is completed, the reaction mixture is stirred at room temperature for 1 hour. Residual citraconic anhydride is removed by addition of 2 M ethanolamine solution, adjusted to pH 9.0.

Cleavage of the modified protected EPO is achieved by addition of the cleavage protease. In case of the construct as described in Example 1(1c) Factor Xa (Roche Molecular Biochemicals, Order No. 602388) is added to the modified EPO 1 : 100 (w/w) and stirred at room temperature for several hours. The progress of cleavage is controlled by taking samples and assaying for cleavage products. In case of to low splitting rate, the amount of the protease can be increased.

In case of applying other proteases, such as IgA protease (prepared as described in EP 513,073) and Genenase^{™} (Genencor Int. Inc.), the procedure is performed accordingly.

The removal of the protease from the reaction mixture is achieved by size exclusion chromatography (SEC) on Superdex™ 75 pg (Pharmacia) or on RP-HPLC.

The Superdex 75 pg material consists of cross-linked agarose and dextran beads. After packing, the column is regenerated with NaOH and equilibrated with a phosphate buffer system with 100 mM sodium chloride.

The reaction mixture from the previous step is concentrated to 10 mg/ml on a Millipore Labscale^{™} TFF System fitted with 10 kDa cut off Millipore Pellicon XL membrane. About 1 - 5 % of the column volume of this solution is applied to the column in one step. The chromatography is performed in the equilibration buffer system. The product is collected in fractions which are pooled according to their purity as analyzed by analytical rpHPLC.

The pooled fractions are concentrated to 7-8 mg/ml on a Millipore Labscale^{™} TFF System fitted with 10 kDa cut off Millipore Pellicon XL membrane.

### EXAMPLE 3: Preparation of N-terminally pegylated EPO

The pegylation reaction is performed at a molar ratio of 1:5 (protected EPO to PEG-SBA reagent) at a final protein concentration of 5 mg/ml. The pegylation reagent used is a methoxy-PEG-SBA, which is a compound of Formula II in which R is methyl; x is 3; and m is from 650 to 750 (average about 730, corresponding to an average molecular weight of about 32 kDa).

The 30 kDa PEG-SBA (Shearwater Polymers, Inc.) is dissolved in 1 mM HCl. Enough 100 mM potassium phosphate buffer, pH 7.5, is added to get a final phosphate concentration of 20 mM in the reaction mixture. Protected EPO (approx. 3 mg/ml in the reaction mixture) is added and the reaction mixture is stirred at ambient temperature (20 - 25°C). After 2 h the reaction is stopped by adjusting the pH to 2.5 with acid.

Decitraconylation of the protein is achieved by stirring the solution at a pH of 2.5 for 5 h at ambient temperature. The reaction is stopped by adjusting the pH to 4.5 with sodium hydroxide and the solution is stored frozen at -20°C until ready for purification.

The separation of N-terminal pegylated EPO (PEG-A1-EPO) from excess reagents, reaction byproducts and non-pegylated EPO is achieved by chromatography on SP-Sepharose FF (Pharmacia). The SP-Sepharose material consists of sulfopropyl (SP)-groups which are covalently bound to the surface of Sepharose beads. The column is filled with SP-Sepharose and regenerated with phosphoric acid and NaOH and equilibrated with a sodium acetate buffer.

The reaction mixture from the previous step is diluted 1:5 with sodium acetate buffer, pH 3 and applied to the SP-Sepharose column. The column is washed with equilibration buffer to remove excess reagents and reaction byproducts. It is followed by washing with 100 mM NaCl. PEG-A1-EPO is then eluted with 200 mM NaCl. The product is collected in fractions which are pooled according to their purity as determined by high performance Size Exclusion Chromatography. Non-pegylated EPO remaining on the column is eluted with 750 mM NaCl.

The PEG-A1 EPO pool is then concentrated to ~ 4.5 - 7.5 mg/ml and diafiltered into the storage buffer, 10 mM potassium phosphate, 100 mM NaCl, pH 7.5. Concentration/Diafiltration was performed with Millipore Labscale^{™} TFF System fitted with 10 kDa cut off Millipore Pellicon XL Biomax membrane at ambient temperature. Concentrated PEG-A1 EPO is sterile filtered and stored frozen at -20°C.

### EXAMPLE 4: In-vivo activity of PEG-A1-EPO determined by the normocythaemic mouse assay

The normocythaemic mouse bioassay is known in the art (Pharm. Europa Spec. Issue Erythropoietin BRP Bio 1997(2)) and a method in the monography of erythropoietin of Ph. Eur. BRP. The samples are diluted with BSA-PBS. Normal healthy mice, 7-15 weeks old, are administered s.c. 0.2 ml of the PEG-A1-EPO-solution from Examples 1-3, EPO solution and buffer as control. Over a period of 6 days, blood is drawn by puncture of the tail vein and diluted such that 1 µl of blood is present in 1 ml of an 0.15 µmol acridine orange staining solution. The staining time is 3 to 10 minutes. The reticulocyte counts are carried out microfluorometrically in a flow cytometer by analysis of the red fluorescence histogram. The reticulocyte counts are given in terms of absolute figures (per 30,000 blood cells analyzed). For the data presented, each group consisted of 5 mice per day, and the mice were bled only once.

The results are shown in Table 1.

The results show the superior activity and the prolonged half life of PEG-A1-EPO species indicated by the significantly increased amounts of reticulocytes and the shift of the reticulocytes count maximum using the same dose per mouse (100 ng), compared to EPO derived from CHO cells.

**Table 1**

| | EPO | PEG-A1-EPO | Control Buffer |
|---|---|---|---|
| 72h | 2404 | 2911 | 857 |
| 96h | 1814 | 3713 | 697 |
| 120h | 901 | not measured | 701 |
| 144h | 536 | 3424 | 708 |

### DESCRIPTION OF THE DRAWINGS

- **Figure 1:**: Primary structure of human EPO (165 amino acids).
- **Figure 2:**: Primary structure of human EPO (166 amino acids).
- **Figure 3:**: Primary structure and corresponding nucleic acid sequence of the APPRIEGR- EPO. The underlined amino acid sequence corresponds to the secretion signal sequence, the wave line to the amino acid sequence specific for the proteolytic cleavage site.

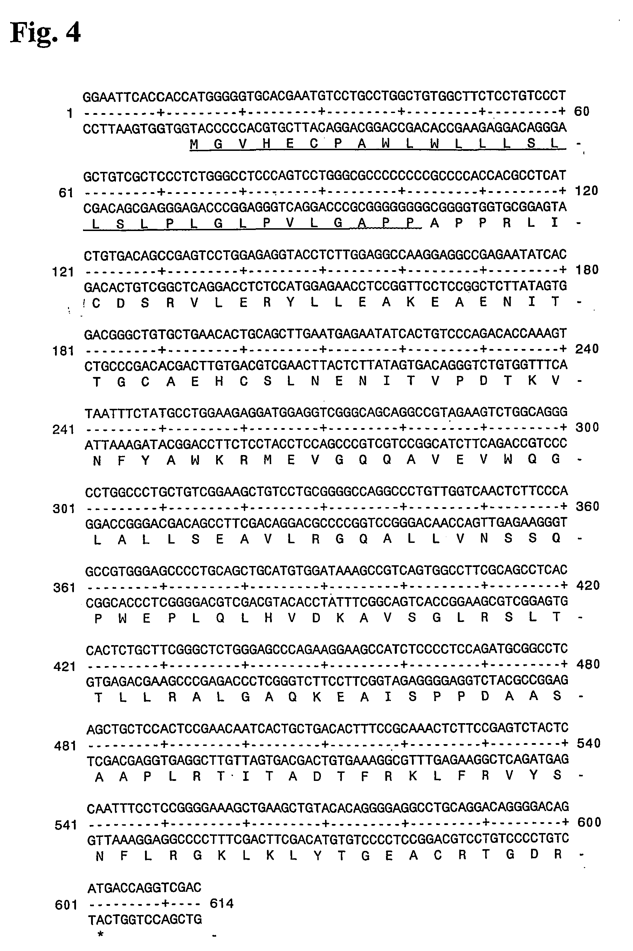

- **Figure 4**:: Primary structure and corresponding nucleic acid sequence of APP-EPO. The underlined amino acid sequence corresponds to the secretion signal sequence, the wave line to the amino acid sequence specific for the proteolytic cleavage site.

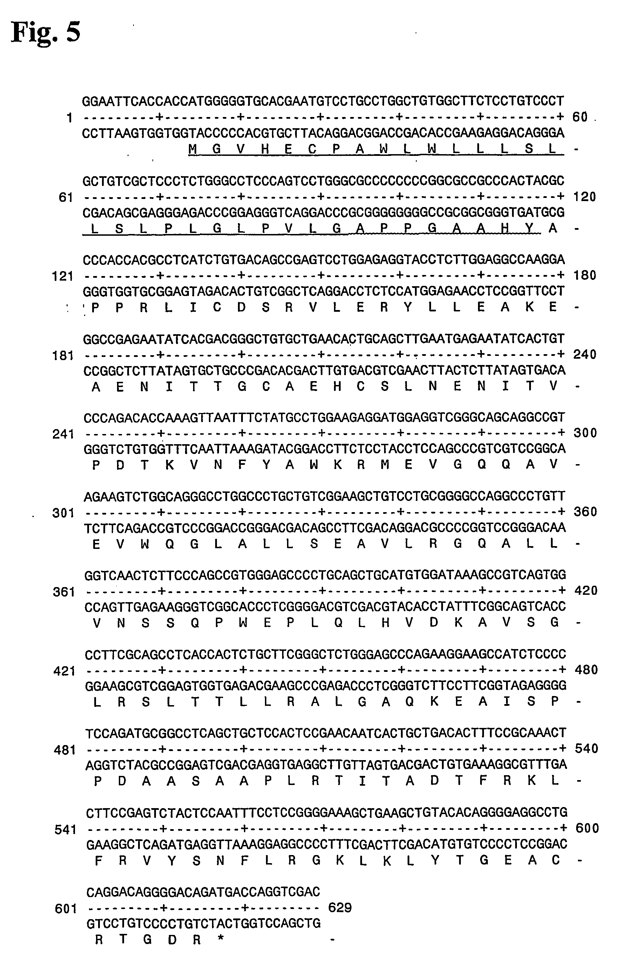

- **Figure 5:**: Primary structure and corresponding nucleic acid sequence of APPGAAHY- EPO. The underlined amino acid sequence corresponds to the secretion signal sequence, the wave line to the amino acid sequence specific for the proteolytic cleavage site.

## Claims

1. A conjugate, said conjugate comprising an erythropoietin glycoprotein having an N-terminal α-amino group and having the *in vivo* biological activity of causing bone marrow cells to increase production of reticulocytes and red blood cells and selected from the group consisting of human erythropoietin and analogs thereof which have sequence of human erythropoietin modified by the addition of from 1 to 6 glycosylation sites or a rearrangement of at least one glycosylation site, wherein the rearrangement comprises a deletion of any of the N-linked carbohydrate sites in human erythropoietin and/or an addition of the N-linked carbohydrate site position 88 of the amino acid sequence of human erythropoietin; said glycoprotein being covalently linked to one poly(ethylene glycol) group of the formula
-CO-(CH₂)ₓ-(OCH₂CH₂)ₘ-OR
with the -CO of the poly(ethylene glycol) group forming an amide bond with said N-terminal α-amino group; wherein
R is a linear or branched alky group having from 1 to 6 carbon atoms;
x is 2 or 3; and
m is from about 450 to about 1350.

2. The conjugate of claim 1, of the formula:
P-NHCO-(CH₂)ₓ-(OCH₂CH₂)ₘ-OR (I)
wherein x, m and R are as defined in claim 1, and P is the residue of the glycoprotein without the N-terminal α-amino group which forms an amide linkage with the poly(ethylene glycol) group.

3. The conjugate of any preceding claim, wherein R is methyl.

4. The conjugate of any preceding claim, wherein m is from 550 to 1000.

5. The conjugate of any preceding claim, wherein m is from about 650 to about 750.

6. The conjugate of any preceding claim, wherein R is methyl and m is from about 650 to about 750.

7. The conjugate of any preceding claim having the formula
CH₃O(CH₂CH₂O)ₘCH₂CH₂CH₂CO-NH-P
wherein m is from 650 to 750 and P is as defined in claim 2.

8. The conjugate of any preceding claim, wherein the glycoprotein is a human erythropoietin.

9. The conjugate according to any of claims 1 to 7, wherein the human erythropoietin glycoprotein is expressed by endogenous gene activation.

10. The conjugate according to any of claims 1 to 9, wherein the glycoprotein has the sequence shown in Fig. 1 or Fig. 2.

11. The conjugate according to any of claims 1 to 8, wherein the glycoprotein has the sequence of human erythropoietin modified by the addition of from 1 to 6 glycosylation sites.

12. The conjugate according to any of claims 1 to 11, wherein the glycoprotein has the sequence of human erythropoietin modified by a modification selected from the group consisting of:
Asn³⁰Thr³²;
Asn⁵¹Thr⁵³,
Asn⁵⁷Thr⁵⁹;
Asn⁶⁹;
Asn⁶⁹Thr⁷¹;
Ser⁶⁸Asn⁶⁹Thr⁷¹;
Val⁶⁷Asn⁸⁸Thr⁹⁰;
Ser⁸⁷Asn⁸⁸Thr⁹⁰;
Ser⁸⁷Asn⁸⁸Gly⁸⁹Thr⁹⁰;
Ser⁸⁷Asn⁸⁸Thr⁹⁰Thr⁹²;
Ser⁸⁷Asn⁸⁸Thr⁹⁰Ala¹⁶²;
Asn⁶⁹Thr⁷¹Ser⁸⁷Asn⁸⁸Thr⁹⁰;
Asn³⁰Thr³²Val⁸⁷Asn⁸⁸Thr⁹⁰;
Asn⁶⁹Ile⁹⁰Thr⁹¹;
Ser⁶⁷Asn⁸⁹Ile⁹⁰Thr⁹¹;
Asn¹³⁶Thr¹³⁸;
Asn¹³⁸Thr¹⁴⁰;
Thr¹²⁵; and
Pro¹²⁴Thr¹²⁵.

13. The conjugate according to any of claims 1 to 7, wherein the glycoprotein has the sequence of human erythropoietin modified by a rearrangement of at least one glycosylation site.

14. The conjugate of claim 13, wherein the rearrangement comprises deletion of any of the N-linked glycosylation sites in human erythropoietin and addition of an N-linked glycosylation site at position 88 of the sequence of human erythropoietin.

15. The conjugate of claim 14, wherein the glycoprotein has the sequence of human erythropoietin modified by a modification selected from the group consisting of:
Gln²⁴ Ser⁶⁷Asn⁸⁸ Thr⁹⁰;
Gln³⁸ Ser⁸⁷ Asn⁸⁸ Thr⁹⁰; and
Gln⁸³ Ser⁸⁷ Asn⁸⁸ Thr⁹⁰.

16. A pharmaceutical composition comprising a conjugate as defined in any of claims 1 to 15 and a pharmaceutically acceptable excipient.

17. Use of a conjugate according to any of claims 1 to 15 for the preparation of medicaments.

18. A process for the preparation of a conjugate as defined in any of claims 1 to 15, comprising
a) expression, and preferably serumfree fermentation, of a recombinant EPO protein comprising a N-terminal peptidic extension which comprises a proteolytic cleavage sequence
b) protecting the ε-amino groups,
c) proteolytic cleavage of the N-terminal peptidic extension,
d) pegylation of the N-terminal α-amino group,
e) deprotection of the ε-amino groups of the erythropoietin glycoprotein,
f) wherein optionally each of the above steps may be followed by a purification step.

19. The process of claim 18 wherein the recombinant EPO comprises a sequence selected from the group consisting of the amino acid sequences shown in Fig. 1 to 5.

20. The process according to claim 18 or 19 wherein in step b) the ε-amino groups are protected by a citraconydation.

21. The process according to any of claims 18 to 20 wherein the N-terminal α-amino group is pegylated with 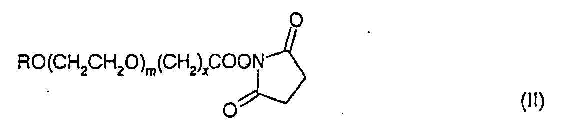 in which R, m and x are as defined in any of claims 1 to 15.

22. Compounds according to any of claims 1 to 15 for use in the treatment of diseases which are associated with anemia in chronic renal failure patients (CRF), AIDS and cancer patients undergoing chemotherapy.

## Patentansprüche

1. Konjugat, wobei das Konjugat ein Erythropoietin-Glycoprotein mit einer N-terminalen α-Aminogruppe umfasst und die *in vivo* biologische Aktivität aufweist, Knochenmarkzellen zu veranlassen, die Produktion von Reticulocyten und roten Blutzellen zu steigern, und ausgewählt ist aus der Gruppe bestehend aus humanem Erythropoietin und Analoga davon, die die Sequenz von humanem Erythropoietin aufweisen, das durch die Addition von 1 bis 6 Glycosylierungsstellen oder durch eine Umlagerung von mindestens einer Glycosylierungsstelle aufweist, wobei die Umlagerung eine Deletion von irgendeinem der N-verknüpften Kohlenhydratstellen in humanem Erythropoietin und/oder einer Addition der N-verknüpften Kohlenhydratstelle Position 88 der Aminosäuresequenz von humanem Erythropoietin, umfasst; wobei das Glycoprotein kovalent an eine Poly(ethylenglycol)gruppe der Formel
-CO-(CH₂)ₓ-(OCH₂CH₂)ₘ-OR
verknüpft ist, wobei das -CO der Poly(ethylenglycol)gruppe eine Amidbindung mit der N-terminalen α-Aminogruppe bilde; wobei
R eine lineare oder verzweigte Alkylgruppe mit von 1 bis 6 Kohlenstoffatomen ist;
x 2 oder 3 ist; und
m von etwa 450 bis etwa 1350 ist.

2. Konjugat nach Anspruch 1 der Formel:
P-NHCO-(CH₂)ₓ-(OCH₂CH₂)ₘ-OR (I)
wobei x, m und R wie in Anspruch 1 definiert sind und P der Rest des Glycoproteins ohne die N-terminale α-Aminogruppe ist, die eine Amidbindung mit der Poly(ethylenglycol)gruppe bildet.

3. Konjugat nach einem vorhergehenden Anspruch, wobei R Methyl ist.

4. Konjugat nach einem vorhergehenden Anspruch, wobei m von 550 bis 1000 ist.

5. Konjugat nach einem vorhergehenden Anspruch, wobei m von etwa 650 bis etwa 750 ist.

6. Konjugat nach einem vorhergehenden Anspruch, R Methyl ist und m von etwa 650 bis etwa 750 ist.

7. Konjugat nach einem vorhergehenden Anspruch mit der Formel
CH₃O(CH₂CH₂O)ₘCH₂CH₂CH₂CO-NH-P
wobei m von 650 bis 750 ist und P wie in Anspruch 2 definiert ist.

8. Konjugat nach einem vorhergehenden Anspruch, wobei das Glycoprotein ein humanes Erythropoietin ist.

9. Konjugat nach einem der Ansprüche 1 bis 7, wobei das humane Erythropoietin Glycoprotein durch endogene Genaktivierung expremiert wird.

10. Konjugat nach einem der Ansprüche 1 bis 9, wobei das Glycoprotein die in Fig. 1 oder Fig. 2 gezeigte Sequenz aufweist.

11. Konjugat nach einem der Ansprüche 1 bis 8, wobei das Glycoprotein die Sequenz von humanem Erythropoietin, das durch die Addition von 1 bis 6
Glycosylierungsstellen modifiziert ist, aufweist.

12. Konjugat nach einem der Ansprüche 1 bis 11, wobei das Glycoprotein die Sequenz von humanem Erythropoietin aufweist, das durch eine Modifizierung ausgewählt aus der Gruppe bestehend aus:
Asn³⁰Thr³²_{;}
Asn⁵¹Thr⁵³;
Asn⁵¹Thr⁵⁹;
Asn⁶⁹;
Asn⁶⁹Thr⁷¹;
Ser⁶⁸Asn⁶⁹Thr⁷¹;
Val⁸⁷Asn⁸⁸Thr⁹⁰;
Ser⁸⁷ Asn⁸⁸Th⁹⁰;
Ser⁸⁷ Asn⁸⁸Gly⁸⁹Thr⁹⁰;
Ser⁸⁷ Asn⁸⁸Thr⁹⁰Thr⁹²;
Ser⁸⁷Asn⁸⁸Thr⁹⁰Ala¹⁶²;
Asn⁶⁹Thr⁷¹Ser⁸⁷ Asn⁸⁸Thr⁹⁰;
Asn³⁰Thr³²Val⁸⁷Asn⁸⁸Thr⁹⁰;
Asn⁸⁹lle⁹⁰Thr⁹¹;
Ser⁸⁷ Asn⁸⁹lle⁹⁰Thr⁹¹;
Asn¹³⁶Thr¹³⁸;
Asn¹³⁸Thr¹⁴⁰;
Thr¹²¹; und
Pro¹²⁴Thr¹²⁵, modifiziert ist

13. Konjugat nach einem der Ansprüche 1 bis 7, wobei das Glycoprotein die Sequenz von humanem Erythropoietin aufweist, das durch eine Umordnung von mindestens einer Glycosylierungsstelle modifiziert ist.

14. Konjugat nach Anspruch 13, wobei die Umordnung die Deletion von irendeinem der N-verknüpften Glycosylierungsstellen in humanem Erythropoietin und Addition von einer N-verknüpften Glycosylierungsstelle an Position 88 der Sequenz von humanem Erythropoietin umfasst.

15. Konjugat nach Anspruch 14, wobei das Glycoprotein die Sequenz von humanem Erythropoietin aufweist, die durch eine Modifikation ausgewählt aus der Gruppe bestehend aus:
Gln²⁴Ser⁸⁷Asn⁸⁸Thr⁹⁰;
Gln³⁸Ser⁸⁷Asn⁸⁸Thr⁹⁰; und
Gln⁸³Ser⁸⁷Asn⁸⁸Thr⁹⁰, modifiziert ist

16. Pharmazeutische Zusammensetzung, umfassend ein Konjugat wie in einem der Ansprüche 1 bis15 definiert und einen pharmazeutisch akzeptablen Hilfsstoff.

17. Verwendung eines Konjugats nach einem der Ansprüche 1 bis 15 zur Herstellung von Medikamenten,

18. Verfahren zur Herstellung eines Konjugats wie in einem der Ansprüche 1 bis 15 definiert, umfassend
a) Expression und bevorzugt serumfreie Fermentation eines rekombinanten EPO-Proteins umfassend eine N-terminale peptidische Verlängerung, die eine proteolytische Spaltungssequenz umfasst
b) Schützen der ε-Aminogruppen,
c) proteolytisches Spalten der N-terminalen peptidischen Verlängerung,
d) Pegylierung der N-terminalen α-Aminogruppe,
e) Entschützen der ε-Aminogruppen des Erythropoietin-Glycoproteins,
f) wobei gegebenenfalls jedem der obigen Schritte ein Aufreinigungsschritt folgen kann.

19. Verfahren nach Anspruch 18, wobei das rekombinante EPO eine Sequenz ausgewählt aus der Gruppe bestehend aus den in Fig. 1 bis 5 gezeigten Aminosäuresequenzen umfasst.

20. Verfahren nach Anspruch 18 oder 19, wobei in Schritt b) die ε-Aminogruppen durch eine Citraconylierung geschützt sind.

21. Verfahren nach einem der Ansprüche 18 bis 20, wobei die N-terminale α-Aminogruppe pegyliert ist mit 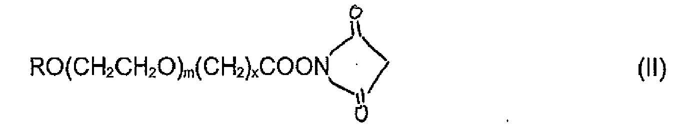 in dem R, m und x definiert sind wie in einem der Ansprüche 1 bis 15.

22. Verbindungen nach einem der Ansprüche 1 bis 15 zur Verwendung bei der Behandlung von Krankheiten, die mit Anämie in chronisch niereninsuffizienten Patienten (CRF), AIDS- und Krebspatienten, die sich einer Chemotherapie unterziehen, einhergehen.

## Revendications

1. Conjugué, ledit conjugué comprenant une glycoprotéine de type érythropoïétine ayant un groupe α-amino N-terminal et ayant l'activité biologique *in vivo* d'induire une augmentation de la production de réticulocytes et de globules rouges sanguins par les cellules de la moelle osseuse, et choisie dans le groupe constitué par l'érythropoïétine humaine et ses analogues qui ont la séquence de l'érythropoïétine humaine modifiée par l'addition de 1 à 6 sites de glycosylation ou le réarrangement d'au moins un site de glycosylation, le réarrangement comprenant une délétion de l'un quelconque des sites d'hydrates de carbone liés à N dans l'érythropoïétine humaine et/ou l'addition d'un site d'hydrate de carbone lié à N en position 88 de la séquence d'aminoacides de l'érythropoïétine humaine; ladite glycoprotéine étant liée par covalence à un groupe polyéthylèneglycol de formule
-CO-(CH₂)ₓ-(OCH₂CH₂)ₘ-OR
le -CO du groupe polyéthylèneglycol formant une liaison amide avec ledit groupe α-amino N-terminal; où
R est un groupe alkyle linéaire ou ramifié de 1 à 6 atomes de carbone;
x est 2 ou 3; et
m est un nombre d'environ 450 à environ 1350.

2. Conjugué selon la revendication 1, de formule:
P-NHCO-(CH₂)ₓ-(OCH₂CH₂)ₘ-OR (I)
dans laquelle x, m et R sont tels que définis dans la revendication 1, et P est le résidu de la glycoprotéine sans le groupe α-amino N-terminal qui forme une liaison amide avec le groupe polyéthylèneglycol.

3. Conjugué selon l'une quelconque des revendications précédentes, dans lequel R est méthyle.

4. Conjugué selon l'une quelconque des revendications précédentes, dans lequel m est un nombre de 550 à 1000.

5. Conjugué selon l'une quelconque des revendications précédentes, dans lequel m est un nombre d'environ 650 à environ 750.

6. Conjugué selon l'une quelconque des revendications précédentes, dans lequel R est méthyle et m est un nombre d'environ 650 à environ 750.

7. Conjugué selon l'une quelconque des revendications précédentes, ayant la formule
CH₃O(CH₂CH₂O)ₘCH₂CH₂CH₂CO-NH-P
dans laquelle m est un nombre de 650 à 750 et P est tel que défini dans la revendication 2.

8. Conjugué selon l'une quelconque des revendications précédentes, dans lequel la glycoprotéine est une érythropoïétine humaine.

9. Conjugué selon l'une quelconque des revendications 1 à 7, dans lequel la glycoprotéine de type érythropoïétine humaine est exprimée par l'activation d'un gène endogène.

10. Conjugué selon l'une quelconque des revendications 1 à 9, dans lequel la glycoprotéine a la séquence présentée sur la figure 1 ou la figure 2.

11. Conjugué selon l'une quelconque des revendications 1 à 8, dans lequel la glycoprotéine a la séquence de l'érythropoïétine humaine modifiée par l'addition de 1 à 6 sites de glycosylation.

12. Conjugué selon l'une quelconque des revendications 1 à 11, dans lequel la glycoprotéine a la séquence de l'érythropoïétine humaine modifiée par une modification choisie dans le groupe constitué par:
Asn³⁰Thr³²;
Asn⁵¹Thr⁵³;
Asn⁵⁷Thr⁵⁹;
Asn⁶⁹;
Asn⁶⁹Thr⁷¹;
Ser⁶⁸ Asn⁶⁹Thr⁷¹;
Val⁸⁷ Asn⁸⁸Thr⁹⁰;
Ser⁸⁷Asn⁸⁸Thr⁹⁰;
Ser¹⁷Asn⁸⁸Gly⁸⁹Thr⁹⁰;
Ser⁸⁷Asn⁸⁸Thr⁹⁰Thr⁹²;
Ser⁸⁷Asn⁸⁸Thr⁹⁰Ala¹⁶²;
Asn⁶⁹Thr⁷¹Ser⁸⁷Asn⁸⁸Thr⁹⁰;
Asn³⁰Thr³²Val⁸⁸Asn⁸⁸Thr⁹⁰;
Asn⁸⁹Ile⁹⁰Thr⁹¹;
Ser⁸⁷Asn⁸⁹Ile⁹⁰Thr⁹¹;
Asn¹³⁶Thr¹³⁸;
Asn¹³⁸Thr¹⁴⁰;
Thr¹²⁵; et
Pro¹²⁴Thr²⁵.

13. Conjugué selon l'une quelconque des revendications 1 à 7, dans lequel la glycoprotéine a la séquence de l'érythropoïétine humaine modifiée par un réarrangement d'au moins un site de glycosylation.

14. Conjugué selon la revendication 13, dans lequel le réarrangement comprend la délétion de l'un quelconque des sites de glycosylation liés à N dans l'érythropoïétine humaine et l'addition d'un site de glycosylation lié à N en position 88 de la séquence de l'érythropoïétine humaine.

15. Conjugué selon la revendication 14, dans lequel la glycoprotéine a la séquence de l'érythropoïétine humaine modifiée par une modification choisie dans le groupe constitué par:
Gln²⁴Ser⁸⁷ Asn⁸⁸Thr⁹⁰;
Gln³⁸Ser⁸⁷Asn⁸⁸Thr⁹⁰; et
Gln⁸³Ser⁸⁷Asn⁸⁸Thr⁹⁰.

16. Composition pharmaceutique comprenant un conjugué tel que défini dans l'une quelconque des revendications 1 à 15 et un excipient pharmaceutiquement acceptable.

17. Utilisation d'un conjugué selon l'une quelconque des revendications 1 à 15 pour la préparation de médicaments.

18. Procédé de préparation d'un conjugué tel que défini dans l'une quelconque des revendications 1 à 15, comprenant:
a) l'expression, et de préférence une fermentation sans sérum, d'une protéine de type EPO recombinée comprenant une extension peptidique N-terminale qui comprend une séquence de coupure protéolytique,
b) la protection des groupes ε-amino,
c) la coupure protéolytique de l'extension peptidique N-terminale,
d) la pégylation du groupe α-amino N-terminal,
e) la déprotection des groupes ε-amino de la glycoprotéine de type érythropoïétine,
f) chacune des étapes précédentes pouvant être suivie d'une étape de purification.

19. Procédé selon la revendication 18, dans lequel l'éryhropoiétine recombinée comprend une séquence choisie dans le groupe constitué par les séquences d'aminoacides présentées sur les figures 1 à 5.

20. Procédé selon la revendication 18 ou 19 dans lequel, dans l'étape b), les groupes ε-amino sont protégés par citraconylation.

21. Procédé selon l'une quelconque des revendications 18 à 20, dans lequel le groupe α-amino N-terminal est pégylé avec 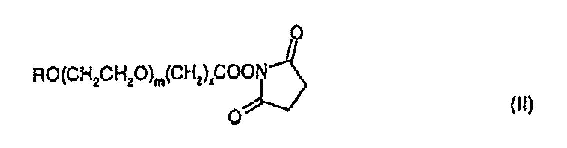 où R, m et x sont tels que définis dans l'une quelconque des revendications 1 à 15.

22. Composés selon l'une quelconque des revendications 1 à 15, pour leur utilisation dans le traitement de maladies qui sont associées à l'anémie chez des patients souffrant d'insuffisance rénale chronique (IRC), des patients souffrant du sida et des patients cancéreux soumis à une chimiothérapie.
